# EUROPEAN PATENT APPLICATION

(11) **EP 4 425 168 A1**
(43) Date of publication of application: **04.09.2024**
(21) Application number: 21961914.5
(22) Date of filing: 29.10.2021
(51) Int. Cl.: G01N 29/024, A61M 16/10

(54) **MEDICAL DEVICE AND OXYGEN CONCENTRATION MEASUREMENT METHOD THEREFOR**

(71) Applicant: Shenzhen Mindray Bio-Medical Electronics Co., Ltd, Shenzhen, Guangdong 518057 (CN)
(72) Inventor: ZHENG, Zhiqiang, Shenzhen, Guangdong 518057 (CN); WU, Lijin, Shenzhen, Guangdong 518057 (CN); AI, Shiming, Shenzhen, Guangdong 518057 (CN); LIU, Jinglei, Shenzhen, Guangdong 518057 (CN)
(74) Representative: KIPA AB
(86) International application number: PCT/CN2021/127587
(87) International publication number: WO 2023/070559

(57) **Abstract**

A medical device (900) and method for measuring an oxygen concentration (200) that is applied to a medical device. The method comprises: acquiring a measurement result of a gas to be measured that is measured by an ultrasonic sensor, wherein the measurement result at least characterizes an oxygen concentration information of the gas to be measured (S210); acquiring a moisture content of the gas to be measured or a moisture content of ambient air, where the medical device is located (S220) , that is measured by an auxiliary sensor; and determining an oxygen concentration of the gas to be measured based on the measurement result measured by the ultrasonic sensor and the moisture content measured by the auxiliary sensor (S230). The method for measuring the oxygen concentration compensates humidity while measuring the oxygen concentration, reduces the influence of the moisture content of the gas to be measured on the measurement value of the ultrasonic sensor, and improves the accuracy while measuring the oxygen concentration.

## Description

### TECHNICAL FIELD

This application relates to the field of medical device, and more specifically to a medical device and method for measuring oxygen concentration that is applied to a medical device.

### BACKGROUND

Current medical devices, such as ventilators, anesthesia machines and medical ceiling support, usually use electrochemical oxygen sensors or paramagnetic oxygen sensors for measuring oxygen concentration. Electrochemical oxygen sensors are consumables with poor signal stability, and would become invalid when the negative electrode material inside such as lead is completely consumed. Therefore, it has shortcomings including necessity of regular calibration and regular replacement. The paramagnetic oxygen sensors have high stability without being bothered by failure caused by electrochemical oxygen consumption. However, they are very sensitive to vibration and not suitable for scenarios like transportation and first aid. In addition, the paramagnetic oxygen sensors are expensive and have high cost.

### SUMMARY

A series of concepts in simplified form are introduced in the Summary, which will be further detailed in Description of Embodiments. The Summary of this invention does not mean to limit the key features and essential technical features of the claimed technical solution, nor does it mean to try to determine the protection scope of the claimed technical solution.

A first aspect of the embodiment of this application provides a method for measuring an oxygen concentration of a ventilator, and the method comprises:
acquiring an initial oxygen concentration of a gas to be measured that is measured by an ultrasonic sensor, wherein the gas to be measured is a patient inhalation gas outputted by the ventilator while performing a respiratory therapy for the patient;
acquiring a moisture content of the gas to be measured or a moisture content of ambient air, where the ventilator is located, that is measured by an auxiliary sensor, wherein the initial oxygen concentration measured by the ultrasonic sensor is affected by the moisture content; and
determining an oxygen concentration of the gas to be measured based on the initial oxygen concentration and the moisture content.

A second aspect of the embodiment of this application provides a method for measuring an oxygen concentration that is to be applied to a medical device, and the method comprises:
acquiring a measurement result of a gas to be measured that is measured by an ultrasonic sensor, wherein the measurement result at least characterizes an oxygen concentration information of the gas to be measured;
acquiring a moisture content of the gas to be measured or a moisture content of ambient air, where the medical device is located, that is measured by an auxiliary sensor; and
determining an oxygen concentration of the gas to be measured based on the measurement result measured by the ultrasonic sensor and the moisture content measured by the auxiliary sensor.

The third aspect of the embodiment of this application provides a medical device, the medical device comprises:
a gas path, used to provide a gas flow path during work;
an ultrasonic sensor, used to measure a gas to be measured in the gas path to obtain a measurement result;
an auxiliary sensor, used to measure a moisture content of the gas to be measured or a moisture content of ambient air where the medical device is located; and
one or more processors, used to:
   acquire the measurement result of the gas to be measured that is measured by the ultrasonic sensor;
   acquire the moisture content of the gas to be measured or the moisture content of ambient air where the medical device is located; and
   determine an oxygen concentration of the gas to be measured based on the measurement result measured by the ultrasonic sensor and the moisture content measured by the auxiliary sensor.

The medical device and the method for measuring oxygen concentration that is applied to a medical device provided by the embodiments of this application compensate humidity while measuring the oxygen concentration, reduce the influence of moisture content in the gas to be measured on the measurement value of the ultrasonic sensor, and improve the accuracy for measuring oxygen concentration.

### BRIEF DESCRIPTION OF THE DRAWINGS

In order to more clearly illustrate the technical solutions in the embodiments of this application, the following will briefly introduce the drawings that need to be used in the description of the embodiments. Obviously, the drawings in the following description are only some embodiments of this invention. For those skilled in the art, other drawings can also be obtained based on these drawings without any creative effort.

In the Figures:
Fig. 1 shows a schematic flow chart of a method for measuring oxygen concentration that is applied to a ventilator according to an embodiment of this application;
Fig. 2 shows a schematic flow chart of a method for measuring oxygen concentration according to an embodiment of this application;
Fig. 3 shows a schematic flow chart of a calibration-based method for measuring oxygen concentration according to an embodiment of this application;
Fig. 4 shows a relationship between the original measurement value of oxygen concentration and the preset value of oxygen concentration by the ultrasonic sensor under different moisture contents according to an embodiment of this application;
Fig. 5 shows a calibration curve according to an embodiment of this application;
Fig. 6 shows a schematic flow chart of a calibration-based method for measuring oxygen concentration according to another embodiment of this application;
Fig. 7 shows a relationship curve between the compensated oxygen concentration and the calibrated oxygen concentration according to an embodiment of this application;
Fig. 8 shows a schematic diagram of a calibration result according to an embodiment of this application;
Fig. 9 shows a schematic block diagram of a medical device according to an embodiment of this application;
Fig. 10 shows a schematic flow chart of a method for monitoring the oxygen concentration according to an embodiment of this application;
Fig. 11 shows a schematic diagram of a device for monitoring the oxygen concentration according to an embodiment of this application;
Fig. 12 shows a schematic diagram of a medical device according to an embodiment of this application.

### DESCRIPTION OF THE EMBODIMENTS

In order to make the objects, technical solutions, and advantages of this application more apparent, exemplary embodiments according to this application will be described in detail below with reference to the drawings. Apparently, the described embodiments are only some of the embodiments of this application, rather than all the embodiments of this application. It should be understood that this application is not limited by the exemplary embodiments described in this application. Based on the embodiments described in this application, all other embodiments obtained by the skilled in the art without creative efforts shall fall within the protection scope of this application.

In the following description, numerous specific details are given in order to provide a more thorough understanding of this application. It will be apparent to one skilled in the art that this application may be practiced without one or more of these details. In other examples, some technical features known in the art are not described in order to avoid confusion with this application.

It should be understood that this application can be embodied in different forms and should not be construed as limited to the embodiments in this application. Rather, these embodiments are provided so that this disclosure will be thorough and complete, and will fully convey the scope of this application to the skilled in the art.

The terminology used herein is for the purpose of describing particular embodiments only and is not intended to be limiting of this application. As used herein, the singular forms "a", "an" and "the" are intended to include the plural forms as well, unless the context clearly dictates otherwise. It should also be understood that the terms "consists of' and/or "comprising", when used in this specification, identify the presence of stated features, integers, steps, operations, elements and/or parts, but do not exclude one or more other presence or addition of features, integers, steps, operations, elements, parts and/or groups. As used herein, the term "and/or" includes any and all combinations of the associated listed items.

In order to thoroughly understand this application, a detailed structure will be provided in the following description to explain the technical solution proposed by this application. Alternative embodiments of this application are described in detail as follows, and this application may have other embodiments besides these detailed descriptions.

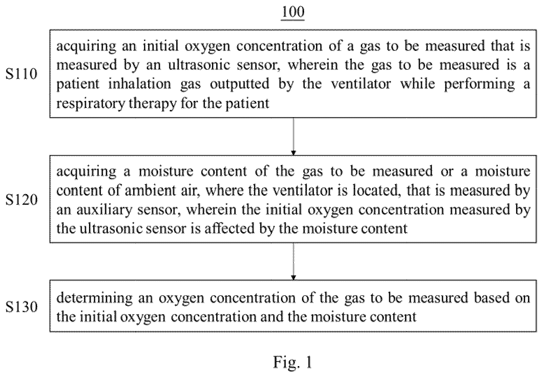

Fig. 1 is a schematic flow chart of a method for measuring oxygen concentration 100 in an embodiment of this application, including the following steps:
In step S 110, an initial oxygen concentration of a gas to be measured that is measured by an ultrasonic sensor is acquired, wherein the gas to be measured is a patient inhalation gas obtained by the ventilator while performing respiratory therapy for the patient;
In step S120, a moisture content of the gas to be measured or a moisture content of ambient air, where the ventilator is located measured by an auxiliary sensor is acquired, wherein the initial oxygen concentration obtained by the ultrasonic sensor is affected by the moisture content;
In step S130, an oxygen concentration of the gas to be measured is determined based on the initial oxygen concentration and the moisture content.

Wherein, the ventilator may be an electric ventilator that uses a turbine to provide an air supply source. The traditional pneumatic ventilator uses high-pressure air source, which is not easy to use, and a separate air cylinder is required for transport. When the turbine is used to provide the air source, the turbine draws air from the surrounding environment of the ventilator and pressurizes it, so no air cylinder is needed. Compared with using the air cylinder as the air source, the turbine cannot provide drying treatment, and the resulting air source contains a certain amount of moisture. Since the measurement results of the ultrasonic sensor are greatly affected by moisture, the moisture in the air source will affect the oxygen concentration measured by the ultrasonic sensor. If the moisture in the air source is removed by adding a drying device inside the ventilator, there will be disadvantages such as increasing the air resistance, reduction of the obtained pressure and regular replacement of the drying device, which has low practical value. The method for measuring oxygen concentration 100 of the embodiment of this application compensates the oxygen concentration based on the moisture content, which can reduce the influence of the moisture content on the measurement value of the ultrasonic sensor, and improve the accuracy for measuring oxygen concentration.

The ultrasonic sensor can be arranged inside the gas circuit of the ventilator, and the gas to be measured is the patient's inhaled gas obtained through the gas circuit when performing respiratory therapy for the patient. For example, the ultrasonic sensor includes a cavity with a gas inlet and a gas outlet on two sides of the cavity. Ultrasonic probes are arranged at the gas inlet and the gas outlet configured to transmit and receive ultrasonic waves. When detecting the oxygen concentration, one ultrasonic probe sends ultrasonic signals, and the other ultrasonic probe receives ultrasonic signals. The time difference between sending ultrasonic signals and receiving ultrasonic signals is the time of flight.

The initial oxygen concentration acquired in step S 110 is the oxygen concentration directly obtained by the ultrasonic sensor. The initial oxygen concentration is obtained by the ultrasonic sensor according to the time of flight and the relationship between the time of flight and oxygen concentration calibrated before leaving the factory. This initial oxygen concentration is inaccurate because the effect of moisture content on the measurement results is not considered when calibrating the ultrasonic sensor.

In step S120, the moisture content of the gas to be measured or the moisture content of ambient air, where the ventilator is located, that is measured by an auxiliary sensor, is acquired.

In one embodiment, the auxiliary sensor comprises a temperature sensor, a humidity sensor and an atmospheric pressure sensor; acquiring a moisture content of the gas to be measured or a moisture content of ambient air, where the ventilator is located, that is measured by an auxiliary sensor, comprises: acquiring a gas temperature measured by the temperature sensor, and determining a saturated vapor pressure according to the gas temperature; acquiring a gas relative humidity measured by the humidity sensor, and determining a partial pressure of moisture based on the gas relative humidity and the saturated vapor pressure; and acquiring an absolute air pressure measured by the atmospheric pressure sensor, and acquiring the moisture content based on the ratio of the partial pressure of moisture to the absolute air pressure. Wherein, the atmospheric pressure sensor may be originally arranged in the ventilator.

In another embodiment, the auxiliary sensor comprises a temperature sensor and a humidity sensor; acquiring a moisture content of the gas to be measured or a moisture content of ambient air where the ventilator is located, that is measured by an auxiliary sensor, comprises: acquiring a gas temperature measured by the temperature sensor, and determining a saturated vapor pressure according to the gas temperature; acquiring a gas relative humidity measured by the humidity sensor, and determining a partial pressure of moisture based on the gas relative humidity and the saturated vapor pressure; and acquiring an absolute air pressure according to the current altitude of the ventilator, or acquiring an absolute air pressure that is preset, and acquiring the moisture content based on the ratio of the partial pressure of moisture to the absolute air pressure. The altitude can be set on the display interface of the ventilator; and the absolute air pressure can also be input by the user.

In an embodiment, at least one of the auxiliary sensors can be arranged inside the gas circuit of the ventilator, and further, at least one of the auxiliary sensors can be integrated with the ultrasonic sensor, that is, arranged inside the cavity of the ultrasonic sensor. In this embodiment, the moisture content obtained based on the measurement value of the auxiliary sensor is the moisture content of the gas to be measured. For example, the temperature sensor, the humidity sensor and the atmospheric pressure sensor directly measure the gas temperature, the gas relative humidity and the absolute pressure of the gas to be measured in the gas circuit of the ventilator respectively. The moisture content determined based on the gas temperature, the gas relative humidity and the absolute pressure is the moisture content of the gas to be measured inside the gas circuit of the ventilator.

In another embodiment, when the air source of the ventilator is the air outside the ventilator, for example, when the turbine is used to extract air from the surrounding environment of the ventilator and pressurize it as the air source, at least one of the auxiliary sensors can be arranged outside the gas circuit of the ventilator and measure the ambient air outside the gas circuit of the ventilator. The moisture content obtained based on the measurement value of the auxiliary sensor is the moisture content in the ambient air where the ventilator is located, that is, the moisture content of air in the gas to be measured. For example, the temperature sensor, the humidity sensor and the atmospheric pressure sensor respectively measure the gas temperature, the gas relative humidity and the absolute air pressure of the air outside the ventilator. The moisture content determined based on the gas temperature, the gas relative humidity and the absolute air pressure is the moisture content of the air outside the ventilator. Since the air source is the air outside the ventilator, the moisture content of the ambient air is the moisture content of the air in the gas to be measured inside the gas circuit; or, through a further algorithm, the moisture content of the gas to be measured inside the gas circuit can be calculated based on the moisture content in the ambient air.

The auxiliary sensor can send the measurement value to the processor of the ventilator or to the processor of the ultrasonic sensor. For example, the temperature sensor, the humidity sensor, and the atmospheric pressure sensor can communicate with the processor of the ultrasonic sensor, and send the measured gas temperature, the gas relative humidity and the absolute air pressure to the processor of the ultrasonic sensor. The processor of the ultrasonic sensor determines the moisture content according to the temperature, the gas relative humidity and the absolute air pressure. Alternatively, the temperature sensor, the humidity sensor, and the atmospheric pressure sensor can communicate with the processor of the ventilator, and send the measured gas temperature, the gas relative humidity and the absolute air pressure to the processor of the ventilator. The processor of the ventilator determines the moisture content according to the temperature, the gas relative humidity and the absolute air pressure. When the auxiliary sensor is integrated into the ultrasonic sensor, the auxiliary sensor and the ultrasonic sensor can share the same processor.

The moisture content acquired in step S 120 may be a relative humidity measured by a humidity sensor; an absolute moisture content measured by a temperature sensor and a humidity sensor; an absolute moisture content measured by a humidity sensor, an atmospheric pressure sensor and a humidity sensor; and a relative humidity preset in the ventilator.

In step S130, the initial oxygen concentration is compensated according to the moisture content to obtain the actual oxygen concentration. Step S130 may be executed by the processor of the ventilator, or by the processor of the ultrasonic sensor, or by both.

For example, firstly, the initial oxygen concentration is obtained according to the measurement data of oxygen concentration, and the initial average relative molecular weight is determined according to the initial oxygen concentration and a preset relationship. Wherein, the preset relationship is related to the environmental condition when calibrating the ultrasonic sensor. The environmental condition when calibrating the ultrasonic sensor can be obtained, and the preset relationship can be obtained according to the environmental condition when calibrating the ultrasonic sensor. The environmental condition during calibration can be the moisture content of the gas to be measured, which is measured by the ultrasonic sensor during calibration. The environmental condition during calibration can also be the gas temperature, the gas relative humidity and the absolute air pressure measured by the temperature sensor, the humidity sensor and the atmospheric pressure sensor during calibration. After acquiring the gas temperature, the gas relative humidity and the absolute air pressure, the moisture content in the gas to be measured during calibration is calculated based on the above data.

Afterwards, the average relative molecular weight of the mixed gas obtained by removing the moisture content is determined according to the moisture content and the initial average relative molecular weight. The initial average relative molecular weight is actually the average relative molecular weight of the mixed gas of air, oxygen and moisture. After excluding the influence of the moisture content on the average relative molecular weight, the average relative molecular weight of the mixed gas obtained by removing the moisture content is determined. The actual oxygen concentration of the gas to be measured can be obtained based on the average relative molecular weight of air, the relative molecular weight of oxygen, and the average relative molecular weight of the mixed gas of air and oxygen.

To sum up, the embodiment of this application provides the method for measuring oxygen concentration 100. When the ultrasonic sensor is used to measure the oxygen concentration, the moisture content is obtained through the auxiliary sensor, and the humidity is compensated according to the moisture content, which can improve the measurement accuracy.

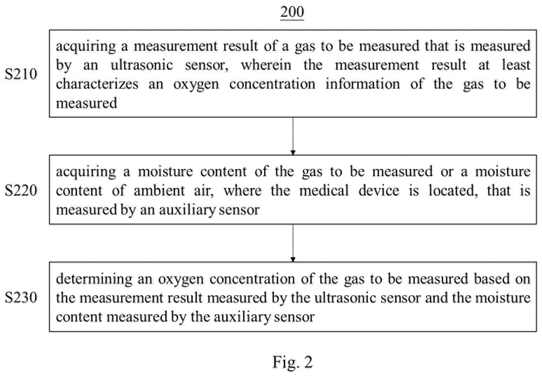

Fig. 2 is a schematic flow chart of a method for measuring oxygen concentration 200 in an embodiment of this application, including the following steps:
In step S210, a measurement result of a gas to be measured that is measured by an ultrasonic sensor is acquired, wherein the measurement result at least characterizes an oxygen concentration information of the gas to be measured;
In step S220, a moisture content of the gas to be measured or a moisture content of ambient air, where the medical device is located, that is measured by an auxiliary sensor, is acquired;
In step S230, an oxygen concentration of the gas to be measured is determined based on the measurement result measured by the ultrasonic sensor and the moisture content measured by the auxiliary sensor.

The method for measuring oxygen concentration 200 of this embodiment is applied to a medical device, such as a ventilator, an anesthesia machine, an oxygen therapy machine, a medical ceiling support, and so on. The medical device can measure the oxygen concentration in the gas delivered to the ventilated object by applying the method for measuring oxygen concentration 200 of the embodiment of this application.

Since electrochemical oxygen device outputs unstable signal and needs to be replaced regularly, while paramagnetic oxygen device is sensitive to vibration and expensive, the embodiment of this application uses an ultrasonic sensor to measure the oxygen concentration, which has the advantages of high output stability, no failure due to consumption, low cost, insensitive to vibration and so on.

Taking ventilator as an example of the medical device, the medical device may be an electric ventilator that uses a turbine to provide an air supply source. The traditional pneumatic ventilator uses high-pressure air source, which is not easy to use, and a separate air cylinder is required for transport. When the turbine is used to provide the air source, the turbine draws air from the surrounding environment of the ventilator and pressurizes it, so no air cylinder is needed. Compared with the air source of the air cylinder, it cannot provide dry treatment measures, and the resulting air source contains a certain amount of moisture. Since the measurement results of the ultrasonic sensor are greatly affected by moisture, the moisture in the air source will affect the oxygen concentration measured by the ultrasonic sensor. If a drying device is provided inside the ventilator to remove the moisture in the air, there will be disadvantages such as increasing the air resistance, reduction of the obtained pressure and regular replacement of the drying device, which has low practical value. The method for measuring oxygen concentration 200 of the embodiment of this application compensates the oxygen concentration based on the moisture content, which can reduce the influence of the moisture content on the measurement value of the ultrasonic sensor, and improve the accuracy for measuring oxygen concentration.

In an embodiment, the measurement result of the gas to be measured that is measured by an ultrasonic sensor acquired in step S210 may be the time of flight measured by the ultrasonic sensor for ultrasonic waves to travel a preset distance in the gas to be measured. When the measurement result is time of flight, step S210 can be executed by the processor of the ultrasonic sensor, that is, the processor of the ultrasonic sensor acquires the time of flight, and determines the oxygen concentration in the gas to be measured according to the time of flight and the moisture content after the moisture content is acquired. Step S210 can also be executed by the processor of the medical device, that is, the ultrasonic sensor sends the measured time of flight to the processor of the medical device, and the processor of the medical device determines the oxygen concentration in the gas to be measured according to the time of flight and the moisture content after the moisture content is acquired. In other embodiments, the processor of the ultrasonic sensor and the processor of the medical device may each process a part of the information to collaboratively obtain the oxygen concentration.

The ultrasonic sensor can be arranged inside the gas circuit of the medical device, and the gas to be measured is the gas in the gas circuit. For example, the ultrasonic sensor includes a cavity with a gas inlet and a gas outlet on two sides of the cavity. Ultrasonic probes are arranged at the gas inlet and the gas outlet configured to transmit and receive ultrasonic waves. When detecting the oxygen concentration, one ultrasonic probe sends ultrasonic signals, and the other ultrasonic probe receives ultrasonic signals. The time difference between sending ultrasonic signals and receiving ultrasonic signals is the time of flight.

The measurement result of the gas to be measured that is measured by an ultrasonic sensor acquired in step S210 may also be the measurement data of oxygen concentration obtained by the ultrasonic sensor, and the initial oxygen concentration can be obtained according to the measurement data of oxygen concentration obtained by the ultrasonic sensor. The initial oxygen concentration is the oxygen concentration directly obtained by the ultrasonic sensor and is affected by the moisture content. The ultrasonic sensor has calibrated the relationship between time of flight and the oxygen concentration before leaving the factory, and the measurement data of oxygen concentration is obtained according to the calibration result. This initial oxygen concentration is inaccurate because the effect of moisture content on the measurement results is not considered when calibrating the ultrasonic sensor.

In step S220, the moisture content of the gas to be measured or the moisture content of ambient air where the medical device is located may be measured based on the auxiliary sensor.

In one embodiment, the auxiliary sensor comprises a temperature sensor, a humidity sensor and an atmospheric pressure sensor , which are used to measure the gas temperature, the gas relative humidity and the absolute pressure respectively; acquiring a moisture content of the gas to be measured or a moisture content of ambient air where the medical device is located based on an auxiliary sensor, comprises: acquiring a gas temperature measured by the temperature sensor, and determining a saturated vapor pressure according to the gas temperature; acquiring a gas relative humidity measured by the humidity sensor, and determining a partial pressure of moisture based on the gas relative humidity and the saturated vapor pressure; and acquiring an absolute air pressure measured by the atmospheric pressure sensor, and acquiring the moisture content based on the ratio of the partial pressure of moisture to the absolute air pressure. Wherein, the atmospheric pressure sensor may be originally arranged in the medical device.

In another embodiment, the auxiliary sensor comprises a temperature sensor and a humidity sensor, which are used to measure the gas temperature and the gas relative humidity respectively. The absolute pressure can be obtained according to the altitude. The altitude can be set on the display interface of the medical device; and the absolute air pressure can also be input by the user. In this embodiment, acquiring a moisture content of the gas to be measured or a moisture content of ambient air where the medical device is located based on an auxiliary sensor, comprises: acquiring a gas temperature measured by the temperature sensor, and determining a saturated vapor pressure according to the gas temperature; acquiring a gas relative humidity measured by the humidity sensor, and determining a partial pressure of moisture based on the gas relative humidity and the saturated vapor pressure; and acquiring an absolute air pressure according to the current altitude of the medical device, or acquiring an absolute air pressure that is preset, and acquiring the moisture content based on the ratio of the partial pressure of moisture to the absolute air pressure.

In an embodiment, at least one of the auxiliary sensors can be arranged inside the gas circuit of the medical device, and further, at least one of the auxiliary sensors can be integrated with the ultrasonic sensor, that is, arranged inside the cavity of the ultrasonic sensor. In this embodiment, the moisture content obtained based on the measurement value of the auxiliary sensor is the moisture content of the gas to be measured. For example, the temperature sensor, the humidity sensor and the atmospheric pressure sensor directly measure the gas temperature, the gas relative humidity and the absolute pressure of the gas to be measured in the gas circuit of the medical device respectively. The moisture content determined based on the gas temperature, the gas relative humidity and the absolute pressure is the moisture content of the gas to be measured inside the gas circuit of the medical device.

In another embodiment, when the air source of the medical device is the air outside the medical device, for example, when the turbine is used to extract air from the surrounding environment of the medical device and pressurize it as the air source, at least one of the auxiliary sensors can be arranged outside the gas circuit of the medical device and measure the ambient air outside the gas circuit of the medical device. The moisture content obtained based on the measurement value of the auxiliary sensor is the moisture content in the ambient air where the medical device is located, that is, the moisture content of air in the gas to be measured. For example, the temperature sensor, the humidity sensor and the atmospheric pressure sensor respectively measure the gas temperature, the gas relative humidity and the absolute air pressure outside the medical device. The moisture content determined based on the gas temperature, the gas relative humidity and the absolute air pressure is the moisture content of the air outside the medical device. Since the air source is the air outside the medical device, the moisture content of the ambient air is the moisture content of the air in the gas to be measured inside the gas circuit; or, through a further algorithm, the moisture content of the gas to be measured inside the gas circuit can be calculated based on the moisture content in the ambient air.

The auxiliary sensor can send the measurement value to the processor of the medical device or to the processor of the ultrasonic sensor. For example, the temperature sensor, the humidity sensor, and the atmospheric pressure sensor can communicate with the processor of the ultrasonic sensor, and send the measured gas temperature, the gas relative humidity and the absolute air pressure to the processor of the ultrasonic sensor. The processor of the ultrasonic sensor determines the moisture content according to the temperature, the gas relative humidity and the absolute air pressure. Alternatively, the temperature sensor, the humidity sensor, and the atmospheric pressure sensor can communicate with the processor of the medical device, and send the measured gas temperature, the gas relative humidity and the absolute air pressure to the processor of the medical device. The processor of the medical device determines the moisture content according to the temperature, the gas relative humidity and the absolute air pressure. When the auxiliary sensor is integrated into the ultrasonic sensor, the auxiliary sensor and the ultrasonic sensor can share the same processor.

In an embodiment, in addition to measuring the moisture content based on the auxiliary sensor, other methods can also be used to acquire the moisture content in the gas to be measured, such as receiving the moisture content measured by other devices through a network or receiving the moisture content input by the user.

In step S230, the oxygen concentration in the gas to be measured is determined based on the measurement result of the ultrasonic sensor and the moisture content. Wherein, when the measurement result of the ultrasonic sensor is the time of flight, the oxygen concentration can be calculated according to the time of flight and the moisture content. When the measurement result of the ultrasonic sensor is the measurement data of oxygen concentration, the actual oxygen concentration can be calculated based on the moisture content and the initial oxygen concentration obtained from the measurement data of oxygen concentration. Step S230 may be executed by the processor of the medical device or by the processor of the ultrasonic sensor, or by both. In some embodiments, it can be considered that the initial oxygen concentration is the measurement result affected by humidity, and the actual oxygen concentration is the measurement result which is not affected by humidity.

For example, when the measurement result is the time of flight, the calculation of the oxygen concentration according to the moisture content and the time of flight includes: determining the average relative molecular weight of the gas to be measured according to the time of flight; determining the oxygen concentration of the gas to be measured according to the average relative molecular weight and moisture content of the gas to be measured.

Specifically, firstly, the transmission distance of the ultrasonic wave and current temperature are known, and the average relative molecular weight of the transmission medium (that is the gas to be measured) of the ultrasonic wave can be calculated according to the time of flight. Since the average relative molecular weight of the gas to be measured has been calculated according to the time of flight, and the moisture content in the air has also been measured in step S220, the average relative molecular weight of the mixed gas obtained by removing the moisture content can be obtained according to the average relative molecular weight and the moisture content of the gas to be measured, and the relative molecular weight of moisture, and then the oxygen concentration can be obtained.

When the measurement result of the ultrasonic sensor is the measurement data of oxygen concentration, the actual oxygen concentration can be calculated in the following way:
Firstly, the initial oxygen concentration is obtained according to the measurement data of oxygen concentration, and the initial average relative molecular weight is determined according to the initial oxygen concentration and a preset relationship. Wherein, the preset relationship is related to the environmental condition when calibrating the ultrasonic sensor. The environmental condition when calibrating the ultrasonic sensor can be obtained, and the preset relationship can be obtained according to the environmental condition when calibrating the ultrasonic sensor. The environmental condition during calibration can be the moisture content of the gas to be measured, which is measured by the ultrasonic sensor during calibration. The environmental condition during calibration can also be the gas temperature, the gas relative humidity and the absolute air pressure measured by the temperature sensor, the humidity sensor and the atmospheric pressure sensor during calibration. After acquiring the gas temperature, the gas relative humidity and the absolute air pressure, the moisture content in the gas to be measured during calibration is calculated based on the above data.

Assuming that the gas to be measured is a dry gas without moisture when calibrating the ultrasonic sensor, for pure air, its oxygen concentration is n1 (21%) with an average relative molecular weight is 28.84; for pure oxygen, its oxygen concentration is n2 (100%) with an average relative molecular weight is 32. Connecting the above two points can obtain a straight line, which is the relationship between the oxygen concentration and the average relative molecular weight. According to this relationship, the initial average relative molecular weight corresponding to the initial oxygen concentration can be obtained. If the gas to be measured is not a pure dry gas when calibrating the ultrasonic sensor, when the oxygen concentration is n1 (21%), the average relative molecular weight of the gas to be measured is less than 28.84 due to the influence of moisture. So, the relationship between the initial oxygen concentration and the initial average relative molecular weight can be adjusted according to the moisture content when calibrating the ultrasonic sensor.

Afterwards, the average relative molecular weight of the mixed gas obtained by removing the moisture content is determined according to the moisture content and the initial average relative molecular weight. The initial average relative molecular weight is actually the average relative molecular weight of the mixed gas of air, oxygen and moisture. After excluding the influence of the moisture content on the average relative molecular weight, the average relative molecular weight of the mixed gas obtained by removing the moisture content is determined. The actual oxygen concentration can be obtained based on the average relative molecular weight of air, the relative molecular weight of oxygen, and the average relative molecular weight of the mixed gas of air and oxygen.

Based on the above description, the method for measuring oxygen concentration 200 of the embodiment of this application can reduce the influence of moisture content on the measurement value of the ultrasonic sensor, and improve the accuracy for measuring oxygen concentration.

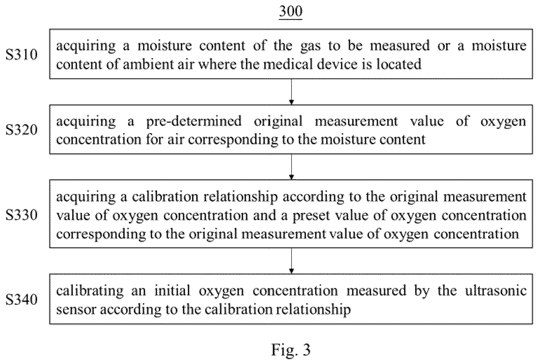

Next, a calibration-based method for measuring oxygen concentration according to an embodiment of this application will be described with reference to Fig. 3. As shown in Fig. 3, the method 300 includes the following steps:
In step S310, the moisture content in the gas to be measured or the moisture content in the ambient air where the medical device is located is acquired;
In step S320, the pre-determined original oxygen concentration corresponding to the moisture content is acquired;
In step S330, a calibration relationship is obtained according to the original oxygen concentration and a preset value of oxygen concentration corresponding to the measurement data of oxygen concentration;
In step S340, the oxygen concentration measured by the ultrasonic sensor is calibrated according to the calibration relationship.

The ultrasonic sensor calibration method 300 of the embodiment of this application can be applied to a medical device, which is a medical device with a ventilation function, such as a ventilator or an anesthesia machine. The medical device can calibrate the ultrasonic sensor by applying the ultrasonic sensor calibration method 300 of the embodiment of this application. The ultrasonic sensor is susceptible to the influence of water vapor due to its measurement principle, and the presence of water vapor will lead to a large deviation in the oxygen concentration measured by the ultrasonic sensor, which can be eliminated by the calibration method 300 of this embodiment.

In step S310, the moisture content in the gas to be measured or the moisture content in the ambient air where the medical device is located may be measured based on the auxiliary sensor to obtain the moisture content of the gas to be measured or the ambient air.

In one embodiment, the auxiliary sensor comprises a temperature sensor, a humidity sensor and an atmospheric pressure sensor; acquiring a moisture content of the gas to be measured based on an auxiliary sensor, comprises: acquiring a gas temperature measured by the temperature sensor, and determining a saturated vapor pressure according to the gas temperature; acquiring a gas relative humidity measured by the humidity sensor, and determining a partial pressure of moisture based on the gas relative humidity and the saturated vapor pressure; and acquiring an absolute air pressure measured by the atmospheric pressure sensor, and acquiring the moisture content based on the ratio of the partial pressure of moisture to the absolute air pressure.

In another embodiment, the auxiliary sensor comprises a temperature sensor and a humidity sensor. The absolute pressure can be obtained according to the altitude. Acquiring a moisture content of the gas to be measured or a moisture content of ambient air, where the medical device is located, that is measured by an auxiliary sensor, comprises: acquiring a gas temperature measured by the temperature sensor, and determining a saturated vapor pressure according to the gas temperature; acquiring a gas relative humidity measured by the humidity sensor, and determining a partial pressure of moisture based on the gas relative humidity and the saturated vapor pressure; and acquiring an absolute air pressure according to the current altitude of the medical device, and acquiring the moisture content based on the ratio of the partial pressure of moisture to the absolute air pressure.

In step S320, a pre-determined original measurement value of oxygen concentration corresponding to the moisture content is acquired. For example, the original measurement value of oxygen concentration is the original measurement value of oxygen concentration in the air. The ultrasonic sensor can be pre-determined when it leaves the factory, and the original measurement value of oxygen concentration of the ambient air where the medical device is located corresponding to the different moisture content in the air is obtained, which is stored in the ultrasonic sensor in a form including a table but not limited thereto.

In step S330, a calibration relationship is obtained according to the original measurement value of oxygen concentration and the preset value of oxygen concentration corresponding to the original measurement value of oxygen concentration. Wherein, the calibration relationship includes a calibration curve, and the calibration curve can be a straight line. For example, when the medical device uses the ultrasonic sensor to measure the oxygen concentration, the ultrasonic sensor needs to be calibrated, and an oxygen concentration C0 measured by the ultrasonic sensor in pure oxygen can be obtained. Subsequently, the medical device obtains the original measurement value of oxygen concentration C1 in the air under the moisture content by, for example, comparing the table stored in the ultrasonic sensor with the actually measured moisture content in the air, and then a straight line is outputted based on the two sets of values (21%, C1) and (100%, C0) to calibrate the ultrasonic sensor.

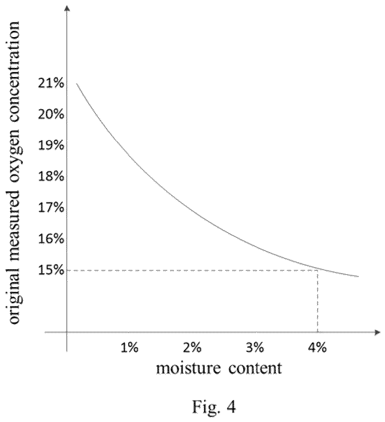

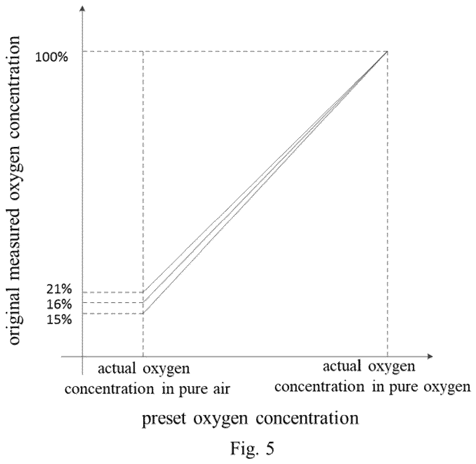

As shown in Fig. 4, the water vapor in the air leads to deviation of the measurement value of the ultrasonic sensor, and the higher the moisture content, the greater the deviation. Assuming that 4% moisture content will cause reduction of the measurement value of the ultrasonic sensor from 21% to 15% when measuring the oxygen concentration of pure air, the processor of the medical device or ultrasonic sensor will obtain the original measurement value of oxygen concentration of air (15%) corresponding to the 4% moisture content through a pre-determined table or curve, which serves as a point with a preset oxygen concentration in air (actual oxygen concentration in pure air, 15%), while the preset value of oxygen concentration and the original measurement value of oxygen concentration in pure oxygen (actual oxygen concentration in pure oxygen, 100%) as another point, so as to obtain a straight line as shown in Fig. 5. It should be noted that the "actual oxygen concentration in pure air" in Fig. 5 can be calculated after knowing the relationship in Fig. 4, because the gas composition in pure air is known. The straight line represents the relationship between the original measurement value of oxygen concentration obtained by the ultrasonic sensor and the preset value of oxygen concentration of the medical device under the current moisture content, so the straight line can be used as a calibration curve to calibrate the initial oxygen concentration measured by the ultrasonic sensor. When the moisture content changes, the calibration curve also changes in real time. Afterwards, in step S340, the initial oxygen concentration measured by the ultrasonic sensor is calibrated according to the above calibration relationship. For example, the initial oxygen concentration measured by the ultrasonic sensor is calibrated to the actual oxygen concentration corresponding to the calibration curve (the horizontal axis in Fig. 5 is the actual oxygen concentration).

Based on the above description, the calibration-based method for measuring oxygen concentration 300 of the embodiment of this application can reduce the influence of the moisture content on the measurement value of the ultrasonic sensor, and improve the accuracy for measuring oxygen concentration.

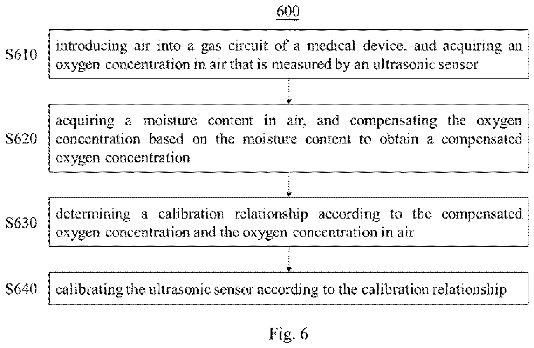

The embodiment of this application also provides another calibration-based method for measuring oxygen concentration. As shown in Fig. 6, the method 600 includes the following steps:
In step S610, air is introduced into the gas circuit of the medical device, and an oxygen concentration measured by an ultrasonic sensor in the air is acquired;
In step S620, a moisture content in the air is acquired, and the oxygen concentration is compensated based on the moisture content to obtain a compensated oxygen concentration;
In step S630, a calibration relationship is determined according to the compensated oxygen concentration and the oxygen concentration in the air;
In step S640, the ultrasonic sensor is calibrated according to the calibration relationship.

The compensated oxygen concentration based on the moisture content can be calibrated again through the method 600, thereby effectively improving the accuracy for measuring oxygen concentration. The ultrasonic sensor is susceptible to the influence of water vapor due to its measurement principle, and the presence of water vapor will lead to a large deviation of the measurement value of the ultrasonic sensor, which can be eliminated by the calibration method of the embodiment of this application.

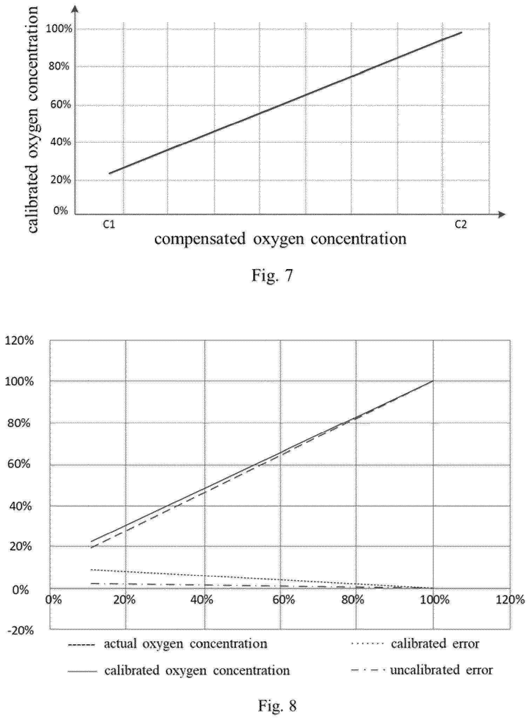

Specifically, air is first introduced into the gas circuit, and the compensated oxygen concentration C1 is calibrated to 21%; then oxygen is introduced into the gas circuit, and the compensated oxygen concentration C2 is calibrated to 100%. After that, see Fig. 7, a straight line is outputted based on the two sets of values (C1, 21%) and (C2, 100%), with the abscissa of the line being the compensated oxygen concentration and the ordinate being the calibrated oxygen concentration. Wherein, compensating the oxygen concentration based on the moisture content may be compensating the original oxygen concentration outputted by the ultrasonic sensor based on the moisture content. In addition, only the oxygen concentration in the air can be calibrated during calibration, that is, the step of introducing oxygen into the gas circuit to calibrate the oxygen concentration of the oxygen can be omitted. The reason why calibrating the oxygen concentration in air is enough is that the ultrasonic sensor has good linearity when measuring oxygen, and the output is very stable in pure oxygen. Fig. 8 shows a schematic diagram of a calibration result according to an embodiment of this application. It can be seen from Fig. 8 that the method 800 of the embodiment of this application can significantly reduce the measurement error of the oxygen concentration.

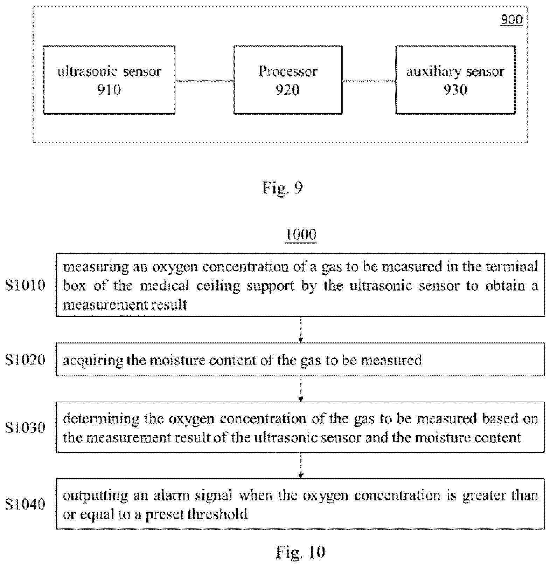

Referring to Fig. 9, the embodiment of this application also provides a medical device, which includes ventilator, anesthesia machine and other medical device with a ventilation function. The medical device is used to replace, control or change the physiological respiration of the ventilated object, and improve the respiratory function of the ventilated object and reduce the respiratory effort of the ventilated object by increasing the lung ventilation. The medical device 900 can be used to implement the method for measuring oxygen concentration 100, the method for measuring oxygen concentration 200, or the method for measuring oxygen concentration 300 described above. The following only describes the main functions of the medical device 900. For other specific details, please refer to the above arts.

As shown in Fig. 9, the medical device 900 includes: a gas circuit for providing a gas flow path during ventilation; an ultrasonic sensor 910 for measuring the gas to be measured in the gas circuit to obtain a measurement result; the auxiliary sensor 930 for measuring the moisture content of the gas to be measured or the moisture content in the ambient air where the medical device is located. One or more processors 920 are used to run executable programs to perform the method for measuring oxygen concentrations as described above. The processor 920 may be the processor of the medical device 900 itself, or the processor of the ultrasonic sensor 910. The processor 920 can be implemented by software, hardware, firmware or any combination thereof, and can use circuits, single or multiple application-specific integrated circuits, single or multiple general-purpose integrated circuits, single or multiple microprocessors, single or multiple programmable logic device, or any combination of the aforementioned circuits and/or devices, or other suitable circuits or devices. The processor 920 can control other components in the medical device 900 or the ultrasonic sensor 910 to perform desired functions.

When the method for measuring oxygen concentration 100 is executed, the processor 920 is used to acquire an initial oxygen concentration of a gas to be measured that is measured by an ultrasonic sensor, wherein the gas to be measured is a patient inhalation gas obtained by the ventilator when performing respiratory therapy for the patient; acquire a moisture content of the gas to be measured or a moisture content of ambient air, where the ventilator is located, that is measured by an auxiliary sensor, wherein the initial oxygen concentration obtained by the ultrasonic sensor is affected by the moisture content; and determine an oxygen concentration of the gas to be measured based on the initial oxygen concentration and the moisture content.

In one embodiment, determining an oxygen concentration of the gas to be measured based on the initial oxygen concentration and the moisture content, comprises: determining an initial average relative molecular weight according to the initial oxygen concentration and a preset relationship; determining an average relative molecular weight of a mixed gas obtained by removing the moisture content from the gas to be measured according to the moisture content and the initial average relative molecular weight; and calculating an actual oxygen concentration of the gas to be measured according to the average relative molecular weight of the mixed gas obtained by removing the moisture content from the gas to be measured.

In one embodiment, the processor 920 is further use to acquire environmental conditions when the ultrasonic sensor 910 is calibrated; and determine the preset relationship according to the environmental conditions when the ultrasonic sensor 910 is calibrated.

In one embodiment, the auxiliary sensor 930 comprises a temperature sensor, a humidity sensor and an atmospheric pressure sensor; acquiring a moisture content of the gas to be measured or a moisture content of ambient air, where the ventilator is located, that is measured by an auxiliary sensor 930, comprises: acquiring a gas temperature measured by the temperature sensor, and determining a saturated vapor pressure according to the gas temperature; acquiring a gas relative humidity measured by the humidity sensor, and determining a partial pressure of moisture based on the gas relative humidity and the saturated vapor pressure; and acquiring an absolute air pressure measured by the atmospheric pressure sensor, and acquiring the moisture content based on the ratio of the partial pressure of moisture to the absolute air pressure.

In one embodiment, the auxiliary sensor comprises a temperature sensor and a humidity sensor; acquiring a moisture content of the gas to be measured or a moisture content of ambient air, where the ventilator is located, that is measured by an auxiliary sensor, comprises: acquiring a gas temperature measured by the temperature sensor, and determining a saturated vapor pressure according to the gas temperature; acquiring a gas relative humidity measured by the humidity sensor, and determining a partial pressure of moisture based on the gas relative humidity and the saturated vapor pressure; and acquiring an absolute air pressure according to the current altitude of the ventilator, or acquiring an absolute air pressure that is preset, and acquiring the moisture content based on the ratio of the partial pressure of moisture to the absolute air pressure.

In one embodiment, the moisture content comprises a relative humidity measured by a humidity sensor; an absolute moisture content measured by a temperature sensor and a humidity sensor; an absolute moisture content measured by a humidity sensor, an atmospheric pressure sensor and a humidity sensor; and a relative humidity preset in the ventilator.

When the method for measuring oxygen concentration 200 is executed, the processor 920 is use to: acquire a measurement result of the gas to be measured that is measured by an ultrasonic sensor 910, wherein the measurement result at least characterizes an oxygen concentration information of the gas to be measured; acquire a moisture content of the gas to be measured or a moisture content of ambient air, where the medical device 900 is located, that is measured by an auxiliary sensor; and determine an oxygen concentration of the gas to be measured based on the measurement result measured by the ultrasonic sensor 910 and the moisture content measured by the auxiliary sensor.

In one embodiment, the measurement result of the gas to be measured that is measured by an ultrasonic sensor 910 comprises measurement data of oxygen concentration obtained by the ultrasonic sensor 910, and the processor 920 is further use to acquire an initial oxygen concentration according to the measurement data of oxygen concentration.

In one embodiment, determining an oxygen concentration of the gas to be measured based on the measurement result of the ultrasonic sensor 910 and the moisture content, comprises: determining an initial average relative molecular weight according to the initial oxygen concentration and a preset relationship; determining an average relative molecular weight of a mixed gas obtained by removing the moisture content from the gas to be measured according to the moisture content and the initial average relative molecular weight; and calculating an actual oxygen concentration of the gas to be measured according to the average relative molecular weight of the mixed gas obtained by removing the moisture content from the gas to be measured.

In one embodiment, the processor 920 is further use to: acquire environmental conditions when the ultrasonic sensor 910 is calibrated; and determine the preset relationship according to the environmental conditions when the ultrasonic sensor 910 is calibrated.

In one embodiment, the measurement result of the gas to be measured that is measured by an ultrasonic sensor 910, comprises: a time of flight for an ultrasonic wave to travel a preset distance in the gas to be measured.

In one embodiment, determining the actual oxygen concentration of the gas to be measured based on the measurement result of the ultrasonic sensor 910 and the moisture content, comprises: determining an average relative molecular weight of the gas to be measured according to the time of flight; and determining the oxygen concentration of the gas to be measured according to the average relative molecular weight of the gas to be measured and the moisture content.

In one embodiment, determining the oxygen concentration of the gas to be measured according to the average relative molecular weight of the gas to be measured and the moisture content, comprises: acquiring an average relative molecular weight of a mixed gas obtained by removing the moisture content from the gas to be measured according to the average relative molecular weight of the gas to be measured and the moisture content; and acquiring the oxygen concentration of the gas to be measured based on the average relative molecular weight of the mixed gas obtained by removing the moisture content from the gas to be measured.

In one embodiment, the auxiliary sensor comprises a temperature sensor, a humidity sensor and an atmospheric pressure sensor; acquiring a moisture content of the gas to be measured or a moisture content of ambient air, where the ventilator is located, that is measured by an auxiliary sensor, comprises: acquiring a gas temperature measured by the temperature sensor, and determining a saturated vapor pressure according to the gas temperature; acquiring a gas relative humidity measured by the humidity sensor, and determining a partial pressure of moisture based on the gas relative humidity and the saturated vapor pressure; and acquiring an absolute air pressure measured by the atmospheric pressure sensor, and acquiring the moisture content based on the ratio of the partial pressure of moisture to the absolute air pressure.

In one embodiment, the auxiliary sensor comprises a temperature sensor and a humidity sensor; acquiring a moisture content of the gas to be measured or a moisture content of ambient air, where the ventilator is located, that is measured by an auxiliary sensor, comprises: acquiring a gas temperature measured by the temperature sensor, and determining a saturated vapor pressure according to the gas temperature; acquiring a gas relative humidity measured by the humidity sensor, and determining a partial pressure of moisture based on the gas relative humidity and the saturated vapor pressure; and acquiring an absolute air pressure according to the current altitude of the medical device, or acquiring an absolute air pressure that is preset, and acquiring the moisture content based on the ratio of the partial pressure of moisture to the absolute air pressure.

When executing the method for measuring oxygen concentration 300, the processor 930 is use to: acquire a pre-determined original measurement value of oxygen concentration corresponding to the moisture content; acquire a calibration relationship according to the original measurement value of oxygen concentration and a preset value of oxygen concentration corresponding to the measurement data of oxygen concentration; and calibrate an oxygen concentration measured by the ultrasonic sensor according to the calibration relationship.

In one embodiment, the original measurement value of oxygen concentration comprises an original measurement value of oxygen concentration by the ultrasonic sensor in air; and the preset value of oxygen concentration corresponding to the measurement data of oxygen concentration comprises an oxygen concentration of air.

In one embodiment, acquiring a calibration relationship according to the original measurement value of oxygen concentration and an preset value of oxygen concentration corresponding to the measurement data of oxygen concentration, comprises: acquiring a straight line by fitting the original measurement value of oxygen concentration and the preset value of oxygen concentration in the air, and the original measurement value of oxygen concentration and the preset value of oxygen concentration in the pure oxygen gas as two points respectively, and using the straight line as the calibration relationship.

Based on the above description, the medical device according to this embodiment can reduce the influence of the moisture content on the measurement value of the ultrasonic sensor, and improve the accuracy for measuring the oxygen concentration.

The embodiment of this application also provides a method for monitoring the oxygen concentration, which can be used in medical gas supply equipment, such as the ceiling support or the ceiling bridge. As shown in Fig. 10, the method for monitoring the oxygen concentration 1000 specifically includes the following steps:
In step S 1010, the oxygen concentration of the gas to be measured in the terminal box of the medical ceiling support is measured by the ultrasonic sensor to obtain the measurement result;
In step S 1020, the moisture content of the gas to be measured is acquired;
In step S 1030, the oxygen concentration of the gas to be measured is determined based on the measurement result of the ultrasonic sensor and the moisture content;
In step S 1040, when the oxygen concentration is greater than or equal to a preset threshold, an alarm signal is outputted.

Medical gas supply equipment can be ceiling support or ceiling bridge, and its main function is to be used as a terminal transfer and equipment platform for medical gas. The ceiling support is mainly suitable for the operating room for terminal transfer of oxygen supply, suction, compressed air, nitrogen and other medical gases in the operating room. The terminal box can be rotated to install the gas interface and gas pressure gauge, and can be provided with power outlet. The ceiling bridge is suitable for ICU of the hospital. It is a medical rescue auxiliary equipment in the modern intensive care unit. It is composed of a bridge frame, a dry area and a wet area, and it is provided with a cantilever structure to increase the space for treatment.

In the traditional medical gas supply equipment, there is no device for monitoring the oxygen concentration inside the terminal box. If for some reasons (such as aging and damage), resulting in the leakage of the oxygen pipeline inside the terminal box, the oxygen concentration inside the terminal box will increase. At this time, if the power socket on the panel of the terminal box is plugged and unplugged, electric sparks may occur, which may cause fires and other situations. In order to prevent the oxygen concentration inside the terminal box from being too high and oxygen leakage, the method for monitoring the oxygen concentration 1000 of the embodiment of this application uses an ultrasonic sensor to measure the oxygen concentration inside the terminal box, and compensates the measurement result based on the moisture content to obtain the accurate oxygen concentration. When it is detected that the oxygen concentration inside the terminal box is greater than the preset threshold, an alarm signal is outputted to remind the user to check and repair the oxygen pipeline in time.

For example, the ultrasonic sensor is arranged inside the terminal box. In one embodiment, the measurement result measured by the ultrasonic sensor is the time of flight for the ultrasonic wave to travel a preset distance in the gas to be measured. Determining the oxygen concentration in the gas to be measured based on the measurement result of the ultrasonic sensor and the moisture content comprises: determining an average relative molecular weight of the gas to be measured according to the time of flight measured by the ultrasonic sensor; determining the proportion of pure oxygen in the gas to be measured according to the average relative molecular weight and moisture content; and determining the oxygen concentration in the gas to be measured according to the proportion of pure oxygen.

In another embodiment, the measurement result measured by the ultrasonic sensor is the original oxygen concentration of the gas to be measured obtained according to the time of flight for the ultrasonic wave to propagate a preset distance in the gas to be measured; Determining the oxygen concentration in the gas to be measured based on the measurement result of the ultrasonic sensor and the moisture content comprises: compensating the original oxygen concentration based on the moisture content to obtain the compensated oxygen concentration.

For example, in step S1020, the moisture content in the gas to be measured may be measured based on the auxiliary sensor. In one embodiment, the auxiliary sensor comprises a temperature sensor, a humidity sensor and an atmospheric pressure sensor; acquiring a moisture content of the gas to be measured that is measured by an auxiliary sensor, comprises: acquiring a gas temperature measured by the temperature sensor, and determining a saturated vapor pressure according to the gas temperature; acquiring a gas relative humidity measured by the humidity sensor, and determining a partial pressure of moisture based on the gas relative humidity and the saturated vapor pressure; and acquiring an absolute air pressure measured by the atmospheric pressure sensor, and acquiring the moisture content based on the ratio of the partial pressure of moisture to the absolute air pressure.

In another embodiment, the auxiliary sensor comprises a temperature sensor and a humidity sensor; acquiring a moisture content of the gas to be measured that is measured by an auxiliary sensor, comprises: acquiring a gas temperature measured by the temperature sensor, and determining a saturated vapor pressure according to the gas temperature; acquiring a gas relative humidity measured by the humidity sensor, and determining a partial pressure of moisture based on the gas relative humidity and the saturated vapor pressure; and acquiring an absolute air pressure according to the altitude, or acquiring a preset fixed absolute air pressure or the absolute air pressure input by the user, and acquiring the moisture content based on the ratio of the partial pressure of moisture to the absolute air pressure.

In addition to measuring the moisture content based on the auxiliary sensor, other methods can also be used to acquire the moisture content in the gas to be measured, such as receiving the moisture content measured by other devices through a network connection, or receiving the moisture content input by the user.

The specific method for determining the oxygen concentration in the measured gas based on the measurement results of the ultrasonic sensor and the moisture content can be described in the method for measuring oxygen concentration 100.

In step S 1040, when the oxygen concentration is greater than or equal to a preset threshold, an alarm signal is outputted. The alarm signal includes but is not limited to the sound alarm signal and the light alarm signal. For example, after outputting the sound and light alarm signal and receiving feedback from the user, the alarm buzzer can be controlled to stop beeping, but the indicator light continues to flash until it is determined that the oxygen concentration in the terminal box is less than the preset threshold, and then the indicator light is controlled to stop blinking.

Based on the above description, the method for monitoring the oxygen concentration according to this embodiment can measure accurate oxygen concentration according to the moisture content, and output an alarm message when the oxygen concentration exceeds a preset threshold, so as to avoid danger caused by oxygen leakage.

The embodiment of this application also provides an device for monitoring the oxygen concentration for implementing the above method for monitoring the oxygen concentration, and the device for monitoring the oxygen concentration includes: an ultrasonic sensor for measuring the oxygen concentration of the gas to be measured in the terminal box of the medical gas supply equipment to obtain a measurement result; a processor for obtaining the moisture content in the gas to be measured; determining the oxygen concentration in the gas to be measured based on the measurement result of the ultrasonic sensor and the moisture content; and when the oxygen concentration is greater than or equal to a preset threshold, outputting an alarm signal. Wherein, the processor may be the processor of the ultrasonic sensor itself, or other processors.

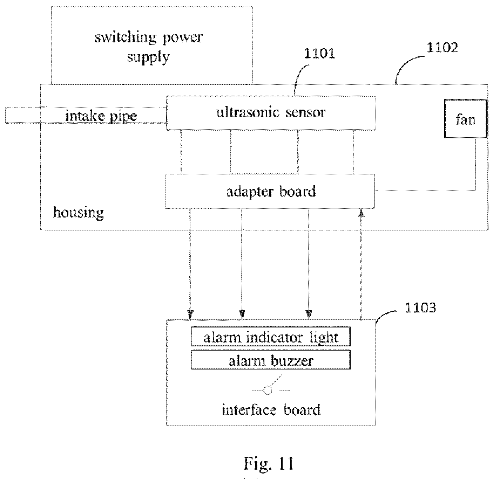

Referring to Fig. 11, it shows an example of the device for monitoring the oxygen concentration of the embodiment of this application. The device for monitoring the oxygen concentration can be arranged inside the terminal box of the medical air supply equipment, which can include a housing 1102 in which the ultrasonic sensor 1101, a fan and an adapter board can be arranged. The ultrasonic sensor 1101 is directly powered from the strong electric socket on the terminal box panel. After the input AC power passes through the switching power supply (such as 24V, 45W switching power supply), +24V DC power is outputted, which then enters the adapter board and is transformed into +5V DC power through DC-DC for the ultrasonic sensor 1101, the auxiliary sensor (not shown in the figure which can be integrated in the ultrasonic sensor 1101), fan, alarm indicator light, alarm buzzer and so on. The gas to be measured (the gas inside the terminal box, mainly a mixture of oxygen and nitrogen) enters the measurement pipeline of the ultrasonic sensor 1101 through the intake pipe under the suction of the fan, so that the ultrasonic sensor 1101 can measure the oxygen concentration of the gas to be measured to obtain the measurement results, and then the gas to be measured is discharged through the fan. The processor of the ultrasonic sensor 1101 obtains the oxygen concentration according to the measurement result and the moisture content, and outputs an alarm signal when it detects that the oxygen concentration is greater than a preset threshold (for example, 28%).

When the alarm device on the HMI (Human Machine Interface) 1103 receives the alarm signal, it starts to alarm. The alarm device may for example be alarm indicator light, alarm buzzer and so on. For example, when the alarm indicator light receives a light alarm signal, it starts to flash at a frequency of 1 Hz. At the same time, when the alarm buzzer receives an acoustic alarm signal, it also sounds intermittently at a frequency of 1 Hz, and the sound frequency of a buzzer may be 2300±500Hz, for example. At this time, the user can press the button on the HMI 1103 to stop the alarm buzzer from sounding, but the alarm indicator light is still flashing. After the oxygen pipeline is repaired, the oxygen concentration in the terminal box will slowly decrease. When the ultrasonic sensor 1101 detects the oxygen concentration is less than 25%, the flashing of the alarm indicator light may be automatically stopped.

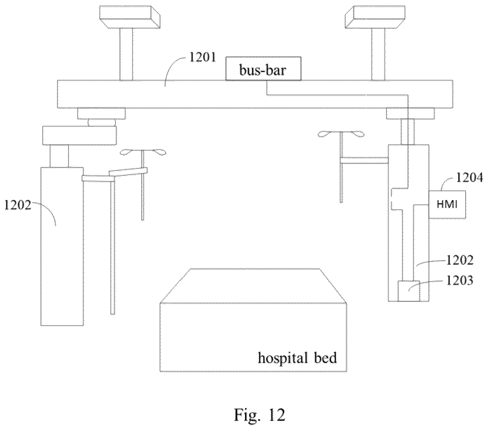

The embodiment of this application also provides a medical gas supply equipment. The medical gas supply equipment includes a terminal box and the above-mentioned device for monitoring the oxygen concentration, and the device for monitoring the oxygen concentration is used to monitor the oxygen concentration in the terminal box. The medical gas supply equipment can be a ceiling support or a ceiling bridge. Fig. 12 shows a ceiling bridge according to an embodiment of this application. As shown in Fig. 12, the ceiling bridge includes a bridge frame 1201, a dry area and a wet area under two sides of the bridge frame 1201. The dry area and the wet area have terminal boxes 1202 respectively, and device for monitoring the oxygen concentrations 1203 are arranged inside the terminal boxes 1202 to monitor the oxygen concentration inside the terminal boxes 1202. The device for monitoring the oxygen concentrations 1203 receive power directly from the strong electric socket on the panel of the terminal box, and the adapter board can also supply power to the alarm device installed on the HMI 1204. In Fig. 12, only the device for monitoring the oxygen concentration 1203 installed inside the terminal box 1202 in the dry area is shown. Similarly, the device for monitoring the oxygen concentration 1203 can also be installed inside the terminal box 1202 in the wet area under a mirrored arrangement.

Based on the above description, the method for monitoring the oxygen concentration, the device for monitoring the oxygen concentration and the medical gas supply equipment according to this embodiment can measure the accurate oxygen concentration according to the moisture content, and output the alarm message when the oxygen concentration exceeds the preset threshold, so as to avoid the danger of leakage. Although example embodiments have been described herein with reference to the accompanying drawings, it should be understood that the above-described example embodiments are exemplary only, and are not intended to limit the scope of this application thereto. Various changes and modifications can be made therein by those of ordinary skill in the art without departing from the scope and spirit of this application. All such changes and modifications are intended to be included within the scope of this application as claimed in the appended claims.

Those of ordinary skill in the art can appreciate that the units and algorithm steps of the examples described in conjunction with the embodiments disclosed herein can be implemented by the electronic hardware, or combination of the computer software and the electronic hardware. Whether these functions are executed by hardware or software depends on the specific application and design constraints of the technical solution. Those skilled in the art may use different methods to implement the described functions for each specific application, but such embodiment should not be regarded as exceeding the scope of this application.

In the several embodiments provided in this application, it should be understood that the disclosed devices and methods may be implemented in other ways. For example, the device embodiments described above are only illustrative. For example, the division of the units is only a logical function division. In actual embodiment, there may be other division methods. For example, multiple units or components can be combined or integrated into another device, or some features may be omitted, or not implemented.

In the description provided herein, numerous specific details are stated. However, it is understood that the embodiments of this application may be practiced without these specific details. In some instances, well-known methods, structures and techniques have not been shown in detail in order not to obscure the understanding of this description.

Similarly, it should be understood that in order to streamline this application and to facilitate understanding of one or more of the various inventive aspects, various features of this application are sometimes grouped together into a single embodiment, figure, or in its description in the description of the exemplary embodiments of this application. However, this method of application is not to be interpreted as reflecting an intention that the claimed application requires more features than are expressly recited in each claim. Rather, as the corresponding claims reflect, the inventive point lies in that the corresponding technical problem may be solved by using less than all features of a single disclosed embodiment. Thus, the claims following this detailed description are hereby expressly incorporated into this detailed description, with each claim standing on its own as a separate embodiment of this application.

It is understood by those skilled in the art that all the features disclosed in this specification (including the accompanying claims, abstract and drawings) and all processes or units of any method or equipment so disclosed may be combined in any combination, except as to the exclusion of the features. Each feature disclosed in this specification (including the accompanying claims, abstract and drawings) may be replaced by alternative features serving the same, equivalent or similar purpose, unless expressly stated otherwise.

In addition, those skilled in the art are able to understand that although some of the embodiments mentioned herein include some features included in other embodiments and not others, the combination of features of different embodiments is meant to fall within the scope of this application and to form different embodiments. For example, in the claims, any one of the claimed embodiments can be used in any combination.

The various component embodiments of this application may be realized in hardware, or in software modules running on one or more processors, or in a combination thereof. Those skilled in the art should understand that a microprocessor or a digital signal processor (DSP) may be used in practice to implement some or all functions of some modules according to the embodiments of this application. This application can also be implemented as an apparatus program (for example, a computer program and a computer program product) for performing a part or all of the methods described herein. Such a program implementing this application may be stored on a computer-readable medium, or may be in the form of one or more signals. Such signals may be downloaded from an Internet site, or provided on the carrier signal, or provided in any other form.

It should be noted that the above-mentioned embodiments illustrate rather than limit this application, and that those skilled in the art will be able to design alternative embodiments without departing from the scope of the appended claims. In the claims, any reference signs placed between parentheses shall not be construed as limiting the claim. This application can be implemented by means of hardware comprising several distinct elements and by means of a suitably programmed computer. In a unit claim enumerating several means, several of these means can be embodied by one and the same item of hardware. The use of the words first, second, third and so on does not indicate any order. These words can be interpreted as names.

The above is only the specific embodiment of this application or the description of the specific embodiment. The scope of protection of this application is not limited thereto. Any technical person familiar with the technical field who can easily think of a change or replacement within the technical scope disclosed in this application shall be covered by the protection of this application. The protection scope of this application should be based on the protection scope of the claims.

## Claims

1. A method for measuring an oxygen concentration of a ventilator, **characterized in that**, the method comprises:
acquiring an initial oxygen concentration of a gas to be measured that is measured by an ultrasonic sensor, wherein the gas to be measured is a patient inhalation gas outputted by the ventilator while performing a respiratory therapy for the patient;
acquiring a moisture content of the gas to be measured or a moisture content of ambient air, where the ventilator is located, that is measured by an auxiliary sensor, wherein the initial oxygen concentration measured by the ultrasonic sensor is affected by the moisture content; and
determining an oxygen concentration of the gas to be measured based on the initial oxygen concentration and the moisture content.

2. The method according to claim 1, **characterized in that**, determining the oxygen concentration of the gas to be measured based on the initial oxygen concentration and the moisture content, comprises:
determining an initial average relative molecular weight according to the initial oxygen concentration and a preset relationship;
determining an average relative molecular weight of a mixed gas, obtained by removing the moisture content from the gas to be measured, according to the moisture content and the initial average relative molecular weight; and
calculating an actual oxygen concentration of the gas to be measured according to the average relative molecular weight of the mixed gas obtained by removing the moisture content from the gas to be measured.

3. The method according to claim 2, **characterized in that**, the method further comprises:
acquiring an environmental condition when the ultrasonic sensor is calibrated; and
determining the preset relationship according to the environmental condition when the ultrasonic sensor is calibrated.

4. The method according to any one of claims 1-3, **characterized in that**, the auxiliary sensor comprises a temperature sensor, a humidity sensor and an atmospheric pressure sensor; and acquiring the moisture content of the gas to be measured or the moisture content of ambient air, where the ventilator is located, that is measured by the auxiliary sensor, comprises:
acquiring a gas temperature that is measured by the temperature sensor, and determining a saturated vapor pressure according to the gas temperature;
acquiring a gas relative humidity that is measured by the humidity sensor, and determining a partial pressure of moisture based on the gas relative humidity and the saturated vapor pressure; and
acquiring an absolute air pressure that is measured by the atmospheric pressure sensor, and acquiring the moisture content based on a ratio of the partial pressure of moisture to the absolute air pressure.

5. The method according to any one of claims 1-3, **characterized in that**, the auxiliary sensor comprises a temperature sensor and a humidity sensor; and acquiring the moisture content of the gas to be measured or the moisture content of ambient air, where the ventilator is located, that is measured by the auxiliary sensor, comprises:
acquiring a gas temperature that is measured by the temperature sensor, and determining a saturated vapor pressure according to the gas temperature;
acquiring a gas relative humidity that is measured by the humidity sensor, and determining a partial pressure of moisture based on the gas relative humidity and the saturated vapor pressure; and
acquiring an absolute air pressure according to a current altitude of the ventilator or acquiring an absolute air pressure that is preset, and acquiring the moisture content based on a ratio of the partial pressure of moisture to the absolute air pressure.

6. The method according to claim 1, **characterized in that**, the moisture content comprises a relative humidity that is measured by a humidity sensor; an absolute moisture content that is measured by a temperature sensor and a humidity sensor; an absolute moisture content that is measured by a humidity sensor, an atmospheric pressure sensor and a humidity sensor; and a relative humidity that is preset in the ventilator.

7. A method for measuring an oxygen concentration that is to be applied to a medical device, **characterized in that**, the method comprises:
acquiring a measurement result of a gas to be measured that is measured by an ultrasonic sensor, wherein the measurement result at least characterizes an oxygen concentration information of the gas to be measured;
acquiring a moisture content of the gas to be measured or a moisture content of ambient air, where the medical device is located, that is measured by an auxiliary sensor; and
determining an oxygen concentration of the gas to be measured based on the measurement result measured by the ultrasonic sensor and the moisture content measured by the auxiliary sensor.

8. The method for measuring the oxygen concentration according to claim 1, **characterized in that**, the measurement result of the gas to be measured that is measured by an ultrasonic sensor comprises measurement data of oxygen concentration outputted by the ultrasonic sensor, and the method further comprises: acquiring an initial oxygen concentration according to the measurement data of oxygen concentration; and
determining an oxygen concentration of the gas to be measured based on the measurement result measured by the ultrasonic sensor and the moisture content measured by the auxiliary sensor, comprises: determining the oxygen concentration of the gas to be measured based on the initial oxygen concentration and the moisture content.

9. The method for measuring the oxygen concentration according to claim 8, **characterized in that**, determining the oxygen concentration of the gas to be measured based on the initial oxygen concentration and the moisture content, comprises:
determining an initial average relative molecular weight according to the initial oxygen concentration and a preset relationship;
determining an average relative molecular weight of a mixed gas obtained by removing the moisture content from the gas to be measured according to the moisture content and the initial average relative molecular weight; and
calculating an actual oxygen concentration of the gas to be measured according to the average relative molecular weight of the mixed gas obtained by removing the moisture content from the gas to be measured.

10. The method for measuring the oxygen concentration according to claim 9, **characterized in that**, the method further comprises:
acquiring an environmental condition when the ultrasonic sensor is calibrated; and
determining the preset relationship according to the environmental conditions when the ultrasonic sensor is calibrated.

11. The method for measuring the oxygen concentration according to claim 1, **characterized in that**, the measurement result of the gas to be measured that is measured by an ultrasonic sensor, comprises: a time of flight for an ultrasonic wave to travel through a preset distance in the gas to be measured; and
determining the actual oxygen concentration of the gas to be measured based on the measurement result measured by the ultrasonic sensor and the moisture content measured by the auxiliary sensor, comprises: determining the actual oxygen concentration of the gas to be measured based on the time of flight and the moisture content.

12. The method for measuring the oxygen concentration according to claim 11, **characterized in that**, determining the actual oxygen concentration of the gas to be measured based on the time of flight and the moisture content, comprises:
determining an average relative molecular weight of the gas to be measured according to the time of flight; and
determining the actual oxygen concentration of the gas to be measured according to the average relative molecular weight of the gas to be measured and the moisture content.

13. The method for measuring the oxygen concentration according to claim 12, **characterized in that**, determining the actual oxygen concentration of the gas to be measured according to the average relative molecular weight of the gas to be measured and the moisture content, comprises:
acquiring an average relative molecular weight of a mixed gas obtained by removing the moisture content from the gas to be measured according to the average relative molecular weight of the gas to be measured and the moisture content; and
acquiring the actual oxygen concentration of the gas to be measured based on the average relative molecular weight of the mixed gas obtained by removing the moisture content from the gas to be measured.

14. The method for measuring the oxygen concentration according to claim 7, **characterized in that**, the auxiliary sensor comprises a temperature sensor, a humidity sensor and an atmospheric pressure sensor; and acquiring the moisture content of the gas to be measured or the moisture content of ambient air where the ventilator is located, that is measured by the auxiliary sensor, comprises:
acquiring a gas temperature that is measured by the temperature sensor, and determining a saturated vapor pressure according to the gas temperature;
acquiring a gas relative humidity that is measured by the humidity sensor, and determining a partial pressure of moisture based on the gas relative humidity and the saturated vapor pressure; and
acquiring an absolute air pressure that is measured by the atmospheric pressure sensor, and acquiring the moisture content based on a ratio of the partial pressure of moisture to the absolute air pressure

15. The method for measuring the oxygen concentration according to claim 14, **characterized in that**, the auxiliary sensor comprises a temperature sensor and a humidity sensor; and acquiring the moisture content of the gas to be measured or the moisture content of ambient air where the ventilator is located, that is measured by the auxiliary sensor, comprises:
acquiring a gas temperature that is measured by the temperature sensor, and determining a saturated vapor pressure according to the gas temperature;
acquiring a gas relative humidity that is measured by the humidity sensor, and determining a partial pressure of moisture based on the gas relative humidity and the saturated vapor pressure; and
acquiring an absolute air pressure according to a current altitude of the medical device or acquiring an absolute air pressure that is preset, and acquiring the moisture content based on a ratio of the partial pressure of moisture to the absolute air pressure.

16. The method for measuring the oxygen concentration according to claim 7, **characterized in that**, the moisture content comprises a relative humidity that is measured by a humidity sensor; an absolute moisture content that is measured by a temperature sensor and a humidity sensor; an absolute moisture content that is measured by a humidity sensor, an atmospheric pressure sensor and a humidity sensor; and a relative humidity that is preset in the medical device.

17. The method for measuring the oxygen concentration according to claim 7, **characterized in that**, the medical device comprises a ventilator, an anesthesia machine, an oxygen therapy machine and a medical ceiling support.

18. The method for measuring the oxygen concentration according to claim 7, **characterized in that**, determining the oxygen concentration of the gas to be measured based on the measurement result measured by the ultrasonic sensor and the moisture content measured by the auxiliary sensor, comprises:
acquiring a pre-determined original measurement value of oxygen concentration for air corresponding to the moisture content;
acquiring a calibration relationship according to the original measurement value of oxygen concentration and a preset value of oxygen concentration corresponding to the original measurement value of oxygen concentration; and
calibrating an initial oxygen concentration measured by the ultrasonic sensor according to the calibration relationship, and acquiring an actual oxygen concentration.

19. The method for measuring the oxygen concentration according to claim 18, **characterized in that**, the original measurement value of oxygen concentration comprises an original measurement value of oxygen concentration that is measured by the ultrasonic sensor in air; and the preset value of oxygen concentration corresponding to the original measurement value of oxygen concentration comprises an oxygen concentration for air.

20. The method for measuring the oxygen concentration according to claim 19, **characterized in that**, acquiring the calibration relationship according to the original measurement value of oxygen concentration and the preset value of oxygen concentration corresponding to the original measurement value of oxygen concentration, comprises:
acquiring a linear relationship by fitting the original measurement value of oxygen concentration and the preset value of oxygen concentration in the air, the original measurement value of oxygen concentration and the preset value of oxygen concentration in pure oxygen respectively, and using the linear relationship as the calibration relationship.

21. A medical device, **characterized in that**, the medical device comprises:
a gas path, used to provide a gas flow path during work;
an ultrasonic sensor, used to measure a gas to be measured in the gas path to obtain a measurement result;
an auxiliary sensor, used to measure a moisture content of the gas to be measured or a moisture content of ambient air where the medical device is located; and
one or more processors, configured to run executable programs to execute the method for measuring the oxygen concentration according to any one of claims 1-20.
